(19) Europäisches Patentamt
European Patent Office
Office européen des brevets

(11) Publication number: **0 153 710**
**A2**

# (12) EUROPEAN PATENT APPLICATION

(21) Application number: 85101936.4

(22) Date of filing: 22.02.85

(51) Int. Cl.[4]: C 12 N 9/66
A 61 K 39/40, A 61 K 39/04
A 61 K 37/54

(30) Priority: 24.02.84 JP 35061/84
24.02.84 JP 35062/84

(43) Date of publication of application:
04.09.85 Bulletin 85/36

(84) Designated Contracting States:
CH DE FR GB LI NL SE

(71) Applicant: Toho Yakuhin Kogyo Kabushiki Kaisha
Yachiyo Building 1-14, Awaji-machi
Higashi-ku Osaka-shi Osaka-fu(JP)

(72) Inventor: Homma, Yuzuru, Dr.
2-34-7, Daizawa
Setagaya-ku Tokyo-to(JP)

(72) Inventor: Morihara, Kazuyuki, Dr.
5-9-2, Hirose Shimamoto-cho
Mishima-gun Osaka-fu(JP)

(74) Representative: Vossius Vossius Tauchner Heunemann Rauh
Siebertstrasse 4 P.O. Box 86 07 67
D-8000 München 86(DE)

**(54) Novel toxoids of elastase of pseudomonas aeruginosa origin.**

(57) The subject matter of the invention are two novel toxoids of elastase of Pseudomonas aeruginosa origin, one of which is obtained by treating a purified elastase produced from Pseudomonas aeruginosa with the synthetic peptide chloroacetyl-N-hydroxy-L-leucyl-L-alanylglycinamide. The other is obtained by treating purified elastase first with formalin and then with the synthetic peptide. The present invention also discloses a method for preparing the toxoids and the use of such toxoids for preventing and treating infections caused by Pseudomonas aeruginosa in human beings and mammalian animals.

EP 0 153 710 A2

Toho Yakuhin Kogyo Kabushiki Kaisha

0153710

# NOVEL TOXOIDS OF ELASTASE OF PSEUDOMONAS AERUGINOSA ORIGIN

This invention relates to the provision of novel toxoids of elastase originating from Pseudomonas aeruginosa by inactivating its virulence, in other words, by inactivating elastase with a synthetic peptide reagent,i.e.chloroacetyl-N-hydroxy-L-leucyl-L-alanylglycinamide while leaving its antigenity as it is.

Pseudomonas aeruginosa is a gram-negative and aerobic bacillus which generally co-exists with a pyogenic bacillus and is known to be a pathogen of diseases as pyothorax, tympanitis, cystitis, hemorrhagic pneumonia in human beings and mammalian animals, especially in minks, which are known to be one of the most expensive sources of furs. In both the fields of human and veterinary medicines, so-called "Opportunistic infections" caused by Pseudomonas aeruginosa have recently provoked attention among physicians as a subject to be solved urgently, and immunotherapy as well as chemotherapy with antibiotics have been used for preventing and treating said infections. However, they are said to be yet incomplete and are under development.

With respect to the above-mentioned immunotherapy, the present inventors have already found that enzymes such as elastase and protease of Pseudomonas aeruginosa origin possess antigenic activity. However, they also possess undesirable activities such as destroying and destructing protein tissues of patients and these undesirable enzymatic activities make infectious diseases caused by Pseudomonas aeruginosa hard to cure.

Then, for the purpose of inactivating these undesirable enzymatic actions while leaving the desirable antigenic actions as they are, elastase and protease have been inactivated with formalin. A vaccin consisting of the above-mentioned two kinds of toxoids and an antigen named OEP, which was derived from Original Endotoxin Protein, which was commonly found in more than 13 kinds of Pseudomonas aeruginosa strains, was prepared. Production, physicochemical properties and immunological properties of the above two kinds of toxoids together with the production of purified crystalline elastase and protease, which were used as a raw material of the toxoids, are disclosed, for example, in US-A-4,160,023. The vaccin is disclosed, for example, in US-A-4,157,389.

This invention relates to toxoids which for the first time have been prepared from purified crystalline elastase obtained from Pseudomonas aeruginosa by treating it with a known synthetic peptide reagent or treating it preliminarily with formalin, and then with the synthetic peptide reagent for inactivating its enzymatic proteinase activity while leaving its antigenic activity as it is. The synthetic peptide reagent employed is chloroacetyl-N-hydroxy-L-leucyl-L-alanylglycinamide represented by a following formula;

$$ClCH_2CO\text{-}N\text{-}HO\text{-}L\text{-}Leu\text{-}L\text{-}Ala\text{-}L\text{-}Gly\text{-}NH_2$$

, wherein Leu, Ala and Gly respectively express leucine, alanine and glycine, which is hereinafter referred to as " Powers Reagent" throughout this specification.

The novel toxoids of this invention are stable in their activity for a longer period of time than the toxoids of elastase which were invented and disclosed by the present inventors in US-A- 4,160,023, and the present toxoids are effective to prevent and treat infectious diseases caused by Pseudomonas aeruginosa both on human beings and mammalian animals.

The afore-mentioned and known toxoids were characterized by the relatively simple producing procedures, which will necessarily result in low production costs. However, the recent clinical experiences have taught that an excess treatment of elastase with formalin intending to positively remove its undesirable proteinase activity, for instance, to the extent of less than 1/100 in potency of that of the original activity, may cause a sudden drop of its desirable activity simultaneously; see table 1.

Table 1

| Sample | Residual Proteinase Activity (%) | Antigenic Activity (μg/ml) |
|---|---|---|
| Untreated | 100 | 30 |
| Treated with HCHO (No.1) | 8 | 30 |
| Treated with HCHO (No.2) | 5.5 | 30 |
| Treated with HCHO (No.3) | 1.5 | 60 |
| Treated with HCHO (No.4) | 0.7 | 240 |
| Treated with HCHO (No.5) | 0.3 | 480 |

Note: In Table 1, antigenic activity is expressed by µg/ml, wherein the numerator indicates the quantity of antigen and the denominator indicates the dilution volume required until a precipitation line will first become observable. So the larger the number is, the less potent is the antigen activity.

It is the object of the present invention to overcome the above-mentioned defects of the known elastase toxoids and to provide stable and effective elastase toxoids. This object is achieved by the invention.

The invention therefore refers to the subject matter contained in the patent claims.

Powers Reagent employed in this invention as an inactivator was at first disclosed by N. Nishino and James C. Powers in Journal of Biological Chemistry (USA), Vol. 225, No. 8, pages 3482 - 3486 (1980), in which Dr. Powers et al. stated that incubation of this reagent with Pseudomonas aeruginosa elastase resulted in a progressive loss of enzyme activity. However, no discussions were made by them about the influence on its antigenic activity.

Physical and chemical properties of Powers Reagent are as follows:

Melting point; 65- 75 °C (Decomposition)

$^1$HNMR spectrum; 9.8 (1H, b, CONCOH), 3.7 (2H, d, NH$\underline{CH_2}$CO), 1.4 (3H, d, $\underline{CH_3}$CH), 0.9 (6H, d, $\underline{CH_3}$CH), 8.2 - 7.9 (2H, m, 2CONH), 7.0 (2H, d, $CONH_2$), 4.4 (2H, $ClCH_2CO$).

One of the products of this invention is a toxoid which is prepared by inactivating Pseudomonas aeruginosa elastase with Powers Reagent, hereinafter referred to as "Toxoid I", and the other product of this invention is a toxoid which is prepared by inactivating Pseudomonas

aeruginosa elastase first with formalin and then with Powers Reagent, hereinafter referred to as "Toxoid II". These toxoids are preparable through procedures conventionally adopted in the field of protein chemistry, and the preparation of purified crystalline elastase therein used as a raw material was disclosed by one of the inventors of this invention, K. Morihara, in the Journal of Biological Chemistry (USA), Vol. 210, pages 3295 - 3304 (1965) in detail (cf. US-A- 4,160,023).

EXAMPLE

Example 1. Preparation of Toxoid I

100 mg of crystalline elastase and 50 mg of Powers Reagent were dissolved in 100 ml of 0.1 M tris-buffer solution (pH 7.0) and the solution was kept for 24 hours long at a room temperature. The reaction mixture was dialyzed against water and the dialysate was lyophilized.

95 mg of toxoid which showed 0.06 mPU/mg protein of a specific activity were obtained.

The meaning of "mPU/mg protein of a specific activity" will be explained later in this specification.

Example 2. Preparation of Toxoid II

100 mg of purified crystalline elastase were dissolved with or without 0.1 M lysine in 0.1 M phosphate buffer solution (pH 7.0) which contained 1 % of formalin and the solution was kept for two days at a room temperature. The reaction mixture was dialyzed against water to remove formalin and lysine, and was concentrated with a collodion film. 22 ml of aqueous solution which were equivalent to 4 mg protein/ml, with a specific activity equal to 3.75 mPU/mg protein

To the thus formed solution, 22 ml of 0.2 M tris-buffer solution (pH 7.0) containing 8 mg of Powers Reagent were added and the mixed solution was kept for 3 days at room temperature and was then dialyzed against water and lyophilized. 80 mg of toxoid having a proteinase activity equivalent to 1/500 of the activity of the original crystalline elastase, were obtained.

Moreover, under the same experimental conditions as the above, the formalin-inactivated elastases were treated with each 2, 1, 0.5 and 0.1 mg, respectively, of Powers Reagent, to obtain 80 - 85 mg of the toxoids, each of which possessed 0.15, 0.2, 0.3 and 0.6 mPU/mg protein of a specific activity.

From the result observed in Example 2, it is recommendable from a practical viewpoint to provide a toxoid which possesses a proteinase activity of less than 1/500 of that of the original crystalline elastase by treating the elastase with 1 % of formalin solution so as to decrease its proteinase activity to 1 - 10 %, preferablly around 5 %, of that of the original elastase and then adding Powers Reagent in a quantity corresponding to the remaining active elastase, wherein 50 mPU correspond to 1 mg of elastase.

Further, comparing above Example 1 with Example 2, it must be noted that the amount of Powers Reagent used for 100 mg of the crystalline elastase is as much as 50 mg in the former and 8 mg in the latter. As Powers Reagent is a relatively expensive substance, Toxoid II is more advantageous regarding the production cost than Toxoid I especially if large scale production is intended.
On the other hand, biological and immunological properties of both are almost equal.

In Examples 1 and 2, the term "mPU/ml protein of a specific activity" signifies the proteinase activity determined by a following procedure;

Casein (2 %, pH 7.4, 1 ml) is admixed with 1 ml of an appropriately diluted solution of an enzyme, and after a reaction at 40 °C for 10 minutes, 2.0 ml of a solution containing 0.1 M trichloroacetic acid and 0.2 M sodium acetate is immediately added for stopping the progress of the reaction. The mixture is kept at the same temperature for 20 minutes to precipitate unreacted casein completely and is filtered. 1 ml of the filtrate is subject to determination of tyrosine contained therein by the Folin's method. Increase of 1 γ of tyrosine per 1 minute indicates a proteinase activity of 1 mPU. 1 mPU x 1,000 is equal to 1 PU.

Physical and chemical properties of Toxoid I have been identified as follows:

(1) Molecular weight; 20,400 (determined by SDS-polyacrylamide gel cataphoresis).

(2) Ultraviolet absorption spectrum; Maximum 280 nm ($E^{280}_{1\%}$ = 14.52, pH 10), Minimum 252 nm.

(3) Isoelectric point; pH 7.0 (determined by a cataphoresis with an acetate film).

(4) Composition of amino acids; Amino acid residues (mol protein) Aspartic acid (15,7), Threonine (6.9), Serine (9.2), Glutamic acid (6.3), Proline (4.5), Glycine (13.5), Alanine ( 11.0), cystine/2 (1.6), Valine (6.7), Methionine (3.0), Isoleucine (3.0), Leucine (5.4), Tyrosine (8.2), Phenylalanine (6.3), Lysine (4.4), Histidine (2.5), Arginine (5.9), Tryptophane (3.0) (Total 117.1 residues).

(5) Color; Colorless powder

(6) Antigenic activity; Positive

(7) Enzymatic activity; Negative

Physical and chemical properties of Toxoid II have been identified as follows:

(1) Molecular weight; 23,300 (determined by SDS-polyacrylamide gel cataphoresis)

(2) Ultraviolet absorption spectrum; Maximum at 280 nm ($E_{1\%}^{280}$ = 11, pH 10), Minimum at 252 nm

(3) Isoelectric point; pH 6.0 (determined by a cataphoresis with an acetate film)

(4) Composition of amino acids; Amino acid residues (mol protein) Aspartic acid (18.1), Threonine (7.9), Serine (10.6), Glutamic acid (7.3), Proline (5.2), Glycine (14.7), Alanine (11.8), Cystine/2 (1.8), Valine (7.8), Methionine (3.5), Isoleucine (3.8), Leucine (6.1), Tyrosine (1.6), Phenylalanine (7.1), Lysine (8.7), Histidine (3.7), Arginine (7.0), Tryptophane (3.5) (Total: 130.2 residues)

(5) Color; Colorless powder

(6) Antigenic activity; Positive

(7) Enzymatic activity; Negative

## EXPERIMENT

Immunological properties of Toxoid I and Toxoid II of this invention will be demonstrated by the following experimental data:

Experiment 1. Antigenicity Test on Rabbits

Method; Three rabbits, each having 2.5 Kg of body weight, were subcutaneously inoculated with each 1 mg of Toxoid I together with Freund's incomplete adjuvant and the inoculation was repeated 3 to 4 times at an interval of 2 weeks. On the day of two weeks after the last inoculation, blood was collected from the rabbits and was availed for following Tests A, B and C. The same method was employed with Toxoid II.

Test A: Passive Hemagglutinative Value Test (PHA Test)

The values were determined according to a method described by one of the present inventors, Y. Homma, in The Japan J. Exp. Med., Vol. 45, No. 5, pages 361 - 365 (1975), and the values were expressed as reciprocal number of a multiplied number of the dilution of sera; see table 2.

Table 2

| PHA Value | | | |
|---|---|---|---|
| Toxoid I | | Toxoid II | |
| (Rabbit No. 1) | 5120 | (Rabbit No.4) | 5110 |
| (Rabbit No. 2) | 2560 | (Rabbit No.5) | 2560 |
| (Rabbit No. 3) | 2560 | (Rabbit No.6) | 2550 |

Note: Before the first inoculation, no elastase PHA value was observed.

Test B: Precipitation in Agar-gel Test

In 0.01 M phosphate buffer-normal saline solution (pH 7.0) which contained 0.1 % of sodium azide, agarose was dissolved to form a concentration of 1.5 % by weight, and 10 ml of the solution were poured in a dish having a diameter of 9 cm and the solution was left to make the agar hard. Several holes were made on the hardened agar and antigens or antisera were separately poured into every hole and the agar was left at 22 °C for 24 hours. Then, existence of a precipitation line was inspected. The concentration of antigens is expressed by a unit of μg/ml, wherein the numerator indicates the quantity of antigens and the denominator indicates the dilution volume which is required until a precipitaion line will first become observable; see table 3.

| Antigen Concentration (µg/ml) | | | |
|---|---|---|---|
| Toxoid I | | Toxoid II | |
| (Rabbit No. 1) | 15 | (Rabbit No. 4) | 15 |
| (Rabbit No. 2) | 15 | (Rabbit No. 5) | 15 |
| (Rabbit No. 3) | 15 | (Rabbit No. 6) | 15 |

Note: The same test as the preceeding employing an authentic crystalline elastase also resulted in a concentration of 15 µg/ml.

Test C: Elastase-neutralizing Activity Test

Sera of the tested rabbits were warmed at 56°C for 30 minutes and 0.2 ml of the sera were added to 0.2 ml of elastase solution, to which normal saline solution was added up to a volume of 2 ml in total. The solution was left for 60 minutes at 37°C. Then, 1 ml of 2 % casein solution was added to 1 ml of the solution. A residual elastase activity was determined with the thus mixed solution. The values of elastase activity neutralizing 1 ml of anti-serum are given in table 4.

Table 4

| Elastase-neutralizing Activity (mPU/ml) | | | |
|---|---|---|---|
| Toxoid I | | Toxoid II | |
| (Rabbit No. 1) | 15 | (Rabbit No. 4) | 15 |
| (Rabbit No. 2) | 12 | (Rabbit No. 5) | 13 |
| (Rabbit No. 3) | 10 | (Rabbit No. 6) | 11 |

Experiment 2. PHA and Elastase-neutralizing Activity on Mice

To a solution containing Toxoid I of this invention in the concentration of 0.5 mg/ml. an equal volume of 1 % potassium alum solution was added. 0.2 ml of

the solution, in which 50 μg of Toxoid I were contained, were inoculated into , of 4 weeks old ddY mice. three times at an interval of 2 weeks. Just before and on the 14th day after the third inoculation, blood was collected from every mouse and values of PHA and elastase-neutralizing activity were inspected in the same manner as with Tests A and C in Experiment 1.

The PHA value was commonly 640 for the first and the second collection of blood and the value of elastase-neutralizing activity was 1.52 mPU/ml for the first collection and 1.83 mPU/ml for the second collection of blood.

Toxoid II of this invention was treated in the same manner as above, resulting in that the PHA value was commonly 640 for the first and the second collection of blood and the value of elastase-neutralizing activity was 1.55 mPU/ml for the first collection and 1.80 mPU/ml for the second collection of blood.

Experiment 3: Antigen Activity on Minks

Two groups, each consisting of twenty or more Sapphire minks, were subcutaneously inoculated with each 500 μg Toxoid I or Toxoid II together with an adjuvant of potassium alum for vaccinization. Two weeks later, each 500 μg of Toxoid I or Toxoid II were inoculated with the adjuvant similarly to the first inoculation. Further, three weeks after the second inoculation, each 1,000 μg of Toxoid I or Toxoid II were subcutaneously inoculated. On the 18th day from the last inoculation, blood was taken from the minks.

In most of sera a 16 to 60 fold increase of the original PHA value was observed.

The results obtained from the experiments confirm that injections of Toxoid I or Toxoid II of this invention result in a prominent elevation of the PHA value and the antigen-neutralizing activity.

With respect to acute toxicity of Toxoid I and Toxoid II of this invention, the $LD_{50}$ value has been determined through intraperitoneal administration on mice to be more than 1 mg/Kg of body weight while $LD_{50}$ of crystalline elastase of Pseudomonas aeruginosa origin is 0.125 mg/Kg of body weight in mice.

Consequently, Toxoid I and Toxoid II of this invention may be used, by themselves or together with pharmaceutically acceptable excipients as a pharmaceutical agent, for the production of anti-bodies or anti-sera, and they are useful for preventing and treating infectious diseases caused by Pseudomonas aeruginosa on human beings and mammalian animals.

The practical use of the toxoids is illustrated by the following experiment: Several minks were divided into two groups. One group was infected with Pseudomonas aeruginosa and the other was kept uninfected. Both groups were treated with a vaccin composed of the following three components: the toxoid of this invention, a separately prepared protease toxoid from Pseudomonas aeruginosa and the above-mentioned OPE (cf. US-A-4,157,389).
The two groups of minks exhibited almost the same survival rate, while a control group of minks which was infected but untreated with the vaccin was found to have no survivals.

The vaccin of the invention will preferably be used for prevention and treatment of chronic infectious diseases caused by Pseudomonas aeruginosa which are generally said to be uncurable by the administration of antibiotics only, and acute Pseudomonas aeruginosa infectious diseases such as burns.

PATENT CLAIMS



1. Toxoid of elastase of Pseudomonas aeruginosa origin, obtainable by treating purified elastase of Pseudomonas aeruginosa for inactivation of its proteinase activity in a buffer solution with the synthetic peptide chloroacetyl-N-hydroxy-L-leucyl-L-alanylglycinamide represented by a following formula;

$$ClCH_2CO-\overset{OH}{\overset{|}{N}}-L-Leu-L-Ala-L-Gly-NH_2$$

wherein Leu, Ala and Gly represent leucine, alanine and glycine, respectively, in an amount of 40 - 60 % by weight of the purified elastase, with subsequent steps of dialysis and lyophilization, said toxoid possessing the following physicochemical properties;

(1) Molecular weight; 20,400 (determined by SDS-polyacrylamide gel cataphoresis).

(2) Ultraviolet absorption spectrum; Maximum at 280 nm ($E_{1\%}^{280}$ = 14.52, pH 10), Minimum at 252 nm.

(3) Isoelectric point; pH 7.0 (determined by cataphoresis with an acetate film).

(4) Composition of amino acids; Amino acid residues (mol protein) Aspartic acid (15.7), Threonine (6.9), Serine (9.2), Glutamic acid (6.3), Proline (4.5), Glycine (13.5), Alanine (11.0), Cystine/2 (1.6), Valine (6.7), Methionine (3.0), Isoleucine (3.0), Leucine (5.4), Tyrosine (8.2), Phenylalanine (6.3), Lysine (4.4), Histidine (2.5), Arginine (5.9), Tryptophane (3.0) [Total 117.1 residues]

(5) Color; Colorless powder.

(6) Antigen activity; Positive.

(7) Enzymatic activity; Negative.

2. Toxoid of elastase of Pseudomonas aeruginosa origin, obtainable by treating purified elastase of Pseudomonas aeruginosa, for inactivation of its proteinase activity in a buffer solution first with formalin to decrease its proteinase activity to the extent of 1 - 10 % of that of the purified elastase and then with the synthetic peptide chloroacetyl-N-hydroxy-L-leucyl-L-alanylglycinamide represented by a following formula;

$$ClCH_2CO-\underset{|}{\overset{OH}{N}}-L-Leu-L-Ala-L-Gly-NH_2$$

, wherein Leu, Ala and Gly represent leucine, alanine and glycine, respectively, to decrease the remaining proteinase activity to the extent of less than 1/500 of that of the purified elastase, with subsequent steps of dialysis and lyophilization, said toxoid possessing the following physicochemical properties;

(1) Molecular weight; 23,300 ( determined by SDS-polyacrylamide gel cataphoresis).

(2) Ultraviolet absorption spectrum; Maximum at 280 nm, ($E_{1\%}^{280}$ = 11, pH 10), Minimum at 252 nm.

(3) Isoelectric point; pH 6.0 (determined by cataphoresis with an acetate film).

(4) Composition of amino acids; Amino acid residues (mol protein) Aspartic acid (18.1), Threonine (7.9), Serine (10.6), Glutamic acid (7.3), Proline (5.2), Glycine (14.7), Alanine (11.8), Cystine/2 (1.8), Valine (7.8), Methionine (3.5), Isoleucine (3.8), Leucine (6.1), Tyrosine (1.6), Phenylalanine (7.1), Lysine (8.7), Histidine (3.7), Arginine (7.0), Tryptophane (3.5) [Total 130.2 residues].

(5) Color; Colorless powder .

(6) Antigen activity; Positive.

(7) Enzymatic activity; Negative.

3. The toxoid according to claim 1, characterized in that the buffer solution used for its production has a pH of about 7.0.

4. The toxoid according to claim 2, characterized in that the buffer solution used for its production has a pH of about 7.0.

5. The toxoid according to claim 1, characterized in that for its production the synthetic peptide is employed in an amount of about 50 % by weight of the purified elastase.

6. The toxoid according to claim 2, characterized in that for its production the proteinase activity is decreased by treating with formalin to 7.5 % of that of the purified elastase and than by treating with the synthetic peptide to 1/500 of that of the purified elastase.

7. An anti-serum for preventing and treating infections caused by Pseudomonas aeruginosa obtainable from human or animal serum by inoculating with the toxoid according to claim 1.

8. An anti-serum for preventing and treating infections caused by Pseudomonas aeruginosa obtainable from human or animal serum by inoculating with the toxoid according to claim 2.

9. The toxoid of claims 1 and 2 for use in preventing and treating infections caused by Pseudomonas aeruginosa.